# FASCICULE DE BREVET EUROPEEN

(11) **EP 2 385 106 B1**
(45) Date de publication et mention de la délivrance du brevet: **09.09.2015**
(21) Numéro de dépôt: 11305524.8
(22) Date de dépôt: 03.05.2011
(51) Int. Cl.: C12N 5/071, A01N 1/02

(54) **Milieu biologique pour la préservation d'une préparation de cellules sécrétrices d'insuline**
Biologisches Medium für die Erhaltung einer Herstellung von Insulin-sekretierenden Zellen
Biological medium for the preservation of a preparation of insulin-secreting cells

(30) Priorité: 05.05.2010 FR 1001936
(43) Date de publication de la demande: 09.11.2011
(73) Titulaire: MACO PHARMA, 59420 Mouvaux (FR); Centre Hospitalier Regional Universitaire de Lille, 59037 Lille Cédex (FR); Université du Droit et de la Santé de Lille 2, 59800 Lille (FR)
(72) Inventeur: Delorme, Bruno, 59700 Marcq-en-Baroeul (FR); Kerr-Conte, Julie, 59000 Lille (FR)
(74) Mandataire: Sayettat, Julien Christian

(56) Documents cités:
- KUMAMOTO S ET AL: "Serum-free culture of insulin-secreting clonal cells from a hamster insulinoma", DIABETES RESEARCH AND CLINICAL PRACTICE, AMSTERDAM, NL LNKD- DOI:10.1016/0168-8227(89)90051-X, vol. 6, no. 1, 3 janvier 1989 (1989-01-03) , pages 1-7, XP023118811, ISSN: 0168-8227 [extrait le 1989-01-03]
- CLARK S A ET AL: "ISLET CELL CULTURE IN DEFINED SERUM-FREE MEDIUM", ENDOCRINOLOGY, vol. 126, no. 4, 1990, pages 1895-1903, XP002090018, ISSN: 0013-7227
- FONG H K ET AL: "Hormones and factors that stimulate growth of a rat islet tumor cell line in serum-free medium.", DIABETES DEC 1981 LNKD- PUBMED:6273246, vol. 30, no. 12, décembre 1981 (1981-12), pages 1022-1028, XP009138318, ISSN: 0012-1797

## Description

L'invention concerne un procédé pour la préservation d'une préparation de cellules sécrétrices d'insuline, destinées à être transplantées à un patient.

L'invention s'applique notamment au domaine de la thérapie cellulaire des pathologies pancréatiques, tel que le diabète, qui vise à préparer des îlots pancréatiques ou îlots de Langerhans à partir de pancréas prélevés sur un donneur en état de mort encéphalique, de coeur arrêté, ou de donneur vivant (c'est-à-dire pancréatectomie partiel ou entier). Dans ce dernier cas, des thérapies de types allogéniques ou autologues peuvent être envisagées. Ces îlots, qui comprennent des cellules bêta sécrétrices d'insuline, sont ensuite transplantés d'un malade receveur dans le but d'obtenir chez ce malade, la restauration de la sécrétion endogène d'insuline.

Ces thérapies cellulaires représentent donc actuellement une alternative intéressante pour traiter les formes les plus sévères du diabète. Un nombre croissant d'équipes propose la pancréatectomie totale chez des patients atteints de TIPMP (Tumeur Intracanalaire Papillaire et Mucineuse de Pancréas). Néanmoins, l'absence de sécrétion endogène d'insuline et de glucagon perturbe profondément le métabolisme de ces patients avec un risque vital à court et à moyen terme. La restauration de l'insulino-secrétion par l'autogreffe des îlots pancréatiques est donc une priorité dans cette pathologie.

Pratiquement, les îlots pancréatiques sont isolés par digestion enzymatique et mécanique à partir d'un pancréas, puis purifiés par gradient de densité. Les îlots sont ensuite transplantés directement au patient ou bien conservés pendant 1 à 3 jours avant leur transplantation.

Cette étape de conservation pré-transplantation s'est avérée bénéfique sur le plan métabolique et immunologique. En effet, le temps passé à conserver les îlots peut permettre de préparer le receveur à la greffe. En outre, cette étape de conservation permet également de réaliser les contrôles qualité sur les îlots nécessaires avant leur perfusion. Enfin, le conditionnement des îlots dans un milieu de conservation permet de les transporter d'un centre à un autre. De façon non surprenante, la culture/conservation des îlots a récemment été adoptée par la plupart des centres de transplantation d'ilots pancréatiques dans le but d'augmenter d'une part la sécurité et d'autre part le coté pratique de la transplantation des îlots.

Cependant, avec les méthodes actuelles de conservation statique, entre 40 et 60% des îlots sont perdus pendant la première période de 24 heures de culture après isolement.

Le document EP-A1-2 119 352 propose un procédé pour conserver les îlots pancréatiques par renouvellement en continu du milieu de culture. Le milieu de culture utilisé dans ce procédé qui fournit les meilleurs résultats est un milieu comprenant le milieu de base CMRL modifié (Cambrex) supplémenté en sérum AB et Stem ease® (Abcys).

Mais l'utilisation du sérum dans le milieu de culture induit un risque en terme d'innocuité puisque sa composition est indéfinie et variable d'un lot à un autre et qu'étant d'origine animale, il existe un risque de contamination, notamment virale, bactérienne et protéique, par exemple par le prion.

Des milieux de culture sans sérum pour la culture et/ou la conservation des îlots pancréatiques ont donc été développés.

Par exemple, le document WO-03/044181 décrit un milieu de culture pour améliorer la fonctionnalité et la viabilité des îlots pancréatiques qui comprend de la vitamine E et de la nicotinamide, et dans lequel le sérum est remplacé par de l'albumine. Le milieu comprend en outre de l'insuline, de la transferrine, du sélénium, de l'acide linoléique, du pyruvate de sodium, du sulfate ou du chlorure de zinc, un tampon HEPES et de la L-glutamine.

D'autres milieux sans sérum pour îlots pancréatiques ont été décrits par Samuel A. Clark dans Endocrinology, 126(4), 1895-1903 (1990). Dans ce document, différents milieux de culture sans sérum sont testés, à savoir des milieux de base sans sérum supplémentés avec une protéose-peptone en tant que substitut de sérum, de la transferrine et une ou plusieurs hormones.

Dans ce même article, la protéose-peptone a ensuite été substituée par l'association d'albumine, d'éthanolamine et de phosphoéthanolamine pour obtenir un milieu défini sans sérum et sans peptone comprenant un milieu de base RPMI-1640 supplémenté avec 0,1 % d'albumine sérique humaine, de la transferrine, l'hormone thyroïdienne (T3), la PRL, l'IGF-I, de l'éthanolamine, de la phosphoéthanolamine, et de la L-glutamine.

Cet article ne décrit pas de milieu comprenant de l'albumine et une peptone. En outre, la peptone est utilisée, au même titre que l'albumine, pour ses propriétés nutritives, en tant que substitut de sérum.

D'autres milieux pour îlots pancréatiques sont décrits dans la littérature. Par exemple, le document WO2005/120576 propose d'ajouter 50 M d'alphatocophérol dans un milieu de culture pour réduire les dommages causés aux îlots pancréatiques par anoxie. Le document US2007/0196810 propose d'ajouter une hémoglobine polymérisée au milieu de culture utilisé pour isoler les îlots du pancréas. Et, dans le document US2006/0246582, un peptide analogue à la chaîne de la laminine A est ajouté au milieu de culture des îlots pour permettre d'augmenter la densité de culture des îlots jusqu'à 300 îlots/mL.

Ces milieux restent cependant insuffisants pour obtenir un taux de survie acceptable des îlots en culture.

Le document WO 2008/009642 décrit un milieu de culture sans sérum destiné à la culture de cellules utilisées notamment pour produire des protéines d'intérêts. Le milieu comprend un milieu de base et une albumine recombinante en tant que substitut de sérum. Le milieu peut en outre comprendre de l'insuline, du sélénite de sodium, de la glutamine, de la transferrine, une peptone, de l'éthanoloamine, de la fétuine, des vitamines, des lipoprotéines, des acides gras et/ou des acides aminés pour améliorer la croissance et la productivité des cellules telles que les hybridomes. Plus spécifiquement, la peptone est combinée avec l'albumine recombinante, l'insuline et éventuellement la transferrine. Dans ce milieu, la peptone est encore utilisée comme élément nutritif pour supporter la croissance et l'expansion des cellules.

L'invention propose un procédé de préservation des cellules insulino-sécrétrices à l'aide d'un milieu biologique sans sérum approuvé pour son application en clinique. Le milieu sans sérum ne contient aucun élément d'origine animale non humaine et atteint les mêmes performances en terme de viabilité cellulaire et fonctionnalité qu'un milieu supplémenté avec du sérum.

A cet effet et selon un premier aspect, l'invention propose un procédé pour la préservation d'une préparation de cellules sécrétrices d'insuline comprenant l'étape de :
- mettre en contact ladite préparation de cellules sécrétrices d'insuline avec un milieu biologique, ledit milieu biologique comprenant un produit nutritif incluant de l'albumine, et comprenant en outre une peptone ou un mélange de peptones en quantité suffisante pour préserver les cellules sécrétrices d'insuline.

Selon un deuxième aspect, l'invention concerne une composition comprenant (a) une préparation de cellules sécrétrices d'insuline et (b) un milieu biologique avec de l'albumine, et une peptone ou un mélange de peptones.

Un autre aspect concerne un médicament pour le traitement des pathologies pancréatiques comprenant une composition de cellules selon le deuxième aspect.

L'invention sera comprise grâce à la description qui suit.
La figure 1 représente le nombre d'îlots équivalents (IE) à J5 dans trois milieux différents.
La figure 2 représente la quantité d'insuline (en µUI / 40 IE/ H) sécrétée par les îlots dans trois milieux différents en présence de 20 mM de glucose.
La figure 3 représente la quantité d'insuline (en µUI / 40 IE/ H) sécrétée par les îlots dans trois milieux différents en présence de 2,8 mM de glucose.
La figure 4 représente l'indice de stimulation (IS) des îlots dans trois milieux différents.
La figure 5 représente la quantité d'insuline intracellulaire par îlots (en µUI / 40 IE) dans trois milieux différents.
La figure 6 représente le dosage de l'apoptose des îlots à 405 nm dans trois milieux différents.

Selon un premier aspect, l'invention concerne un procédé de préservation d'une préparation de cellules sécrétrices d'insuline. Notamment, le procédé permet de conserver les cellules à court terme, c'est-à-dire de l'ordre de 1 à 7 jours, en particulier de 1 à 5 jours à 37°C ou à 22-25°C.

Selon l'invention, le procédé de préservation est un procédé qui protège les cellules sécrétrices d'insuline des effets délétères de leur environnement, en maintenant leur viabilité et leur fonctionnalité.

L'invention concerne également en deuxième aspect une composition comprenant une préparation de cellules sécrétrices d'insuline et un milieu biologique comprenant de l'albumine et une peptone ou un mélange de peptones.

La composition de l'invention est capable de maintenir en bon état les cellules sécrétrices d'insuline vivantes et potentiellement de les protéger des effets délétères d'un prélèvement, d'un isolement et d'une greffe.

La préparation de cellules sécrétrices d'insuline est en particulier une préparation des d'îlots pancréatiques ou une préparation d'autres types de cellules différenciées en cellules sécrétrices d'insuline, comme les cellules souches adultes (pancréatiques, mésenchymateuses, hématopoïétiques, cellules reprogrammées en cellules souches pluripotentes (iPS), ...).

Notamment, la préparation de cellules sécrétrices d'insuline est une préparation d'îlots pancréatiques issus d'un pancréas d'un donneur en état de mort encéphalique. Les îlots sont isolés par digestion enzymatique et mécanique du pancréas puis purifiés par gradient de densité. Ils sont ensuite conservés avant d'être transplantés.

Le procédé et la composition de l'invention s'appliquent aux îlots ainsi isolés contenus dans des préparations de haute pureté, notamment de l'ordre de 90%, de moyenne pureté, mais aussi à des préparations de pureté moindre telle qu'inférieure à 50%.

Ainsi, avec un tel procédé d'isolement, la préparation de cellules sécrétrices comprend en outre des enzymes protéolytiques, sécrétées par les cellules et tissus exocrines du pancréas.

Le procédé de préservation et la composition de l'invention permettent de maintenir la fonctionnalité et la viabilité des cellules sécrétrices d'insuline. Notamment, les cellules ainsi préservées gardent leur capacité à sécréter de l'insuline. Le procédé et la composition de l'invention ne visent pas prioritairement à expandre ou multiplier les îlots pancréatiques mais plutôt à conserver leur capacité à exprimer l'insuline.

Selon le procédé de l'invention, la préparation de cellules sécrétrices d'insuline est mise en contact avec un milieu biologique comprenant d'une part un produit nutritif incluant de l'albumine et d'autre part une peptone ou un mélange de peptones en quantité suffisante pour préserver les cellules sécrétrices d'insuline.

Il résulte de ce procédé une composition comprenant une préparation de cellules sécrétrices d'insuline et un milieu biologique comprenant d'un part un produit nutritif incluant de l'albumine et d'autre part une peptone ou un mélange de peptones. Cette composition permet de préserver les cellules sécrétrices d'insuline.

La concentration des îlots pancréatiques dans le milieu biologique est comprise entre 400 et 40 000 îlots équivalents/ mL. Avantageusement, la concentration des îlots est de l'ordre de 500-3 000 îlots équivalents /mL.

Du fait que les îlots peuvent avoir des tailles très différentes, on parle d'îlots équivalents (IE), un îlot équivalent correspondant à un îlot standard de 150 µm de diamètre.

En particulier, aucun milieu biologique, produit nutritif et milieu de culture ne contient de sérum.

Selon un mode de réalisation, le produit nutritif comprend un milieu de culture liquide sans sérum, supplémenté en albumine.

Le milieu biologique comprend par exemple un milieu de culture de base de type CMRL 1066 (In Vitrogen) ou MEM (MacoPharma) ou Ham's F10, ou M199.

De façon avantageuse, l'albumine est de l'albumine de sérum, en particulier de l'albumine de sérum humain ou de l'albumine recombinante humaine. Elle remplace le sérum utilisé dans les milieux de culture à usage de recherche.

L'albumine est une protéine qui favorise le transport des molécules lipidiques (acides gras) et/ ou liposolubles (notamment des vitamines), et de quelques ions métalliques (Cu2+, Zn2+ et Ca2+).

Dans un milieu de culture sans sérum, l'albumine joue un rôle dans le support de la croissance cellulaire de divers types de cellules, essentiellement lorsque complexée à une fraction lipidique (acides gras).

Pour permettre la préservation des cellules sécrétrices d'insuline, le milieu biologique comprend entre 0,1 et 1% en poids/volume d'albumine, notamment 0,625 % en poids/volume.

En outre, le milieu biologique comprend une peptone ou un mélange de peptones.

Une peptone est une substance protidique soluble, qui résulte de l'hydrolyse chimique ou enzymatique de protéines. Les peptones sont obtenues à partir des protéines du lait telle que la caséine, de protéines animales issues des muscles ou des organes, de levures, ou de protéines végétales.

Avantageusement, la peptone utilisée dans le procédé et la composition de l'invention est d'origine non animale pour éviter les risques liés aux protéines animales, comme les encéphalopathies spongiformes bovines ou transmissibles.

Par exemple, la peptone préférée est une peptone végétale produite à partir de plantes. En particulier, la peptone est une peptone de pois, de blé, de pomme de terre, de riz, de coton ou de soja.

Avec les procédés de préparation des îlots, les préparations des îlots ne sont pas pures et contiennent encore jusqu'à 50 % de cellules exocrines telles que des cellules épithéliales. Ces cellules exocrines contiennent différentes préproenzymes protéolytiques qui se transforment en enzymes protéolytiques actives telles que la trypsine, la chymotrypsine et la carboxypeptidase, susceptibles d'endommager les îlots pancréatiques et d'affecter leur fonctionnalité et leur viabilité.

La peptone contient des acides aminés, des peptides, des vitamines et des éléments essentiels.

Dans le milieu biologique, la peptone constitue ainsi une cible alternative pour ces enzymes protéolytiques, qui sont alors moins susceptibles d'attaquer les îlots pancréatiques.

Dans le procédé et la composition de l'invention, les peptones constituent alors un substrat pour les enzymes protéolytiques des tissus et/ou cellules exocrines.

Le milieu biologique comprend notamment entre 0,01 et 10 % en poids/volume de peptone, particulièrement entre 0,05 et 5 % en poids/volume de peptone et plus particulièrement entre 0,1% et 1% en poids/volume de peptone. Par exemple, la concentration de peptone dans le milieu est de 0,1 % en poids/volume.

Cette concentration est suffisante pour améliorer la survie et la capacité de sécrétion d'insuline des cellules sécrétrices d'insuline.

En outre, il a été montré qu'une concentration importante d'albumine, notamment de l'ordre de 5% en poids/volume permet de réduire l'apoptose dans des îlots humains conservés in vitro (Artif Cells Blood Substit Immobil Biotechnol. 2008;36(1):74-81).

Selon l'invention, la présence d'une peptone dans le milieu biologique permet de réduire l'apoptose des îlots avec une concentration relativement faible d'albumine, par exemple inférieure à 1% en poids/volume d'albumine.

D'autre part, l'albumine est également une cible de la trypsine et de la chymotrypsine. Elle renforce ainsi le rôle qu'on attribue à la peptone, de faire diversion aux enzymes protéolytiques du pancréas.

Avantageusement, la peptone utilisée dans le procédé de l'invention est riche en protéines. Notamment, elle comprend plus de 70% en poids, de préférence plus de 80% en poids de protéines.

La teneur en protéines de la peptone est obtenue en multipliant par 6,25 la teneur en azote protéique de la peptone, elle-même calculée en utilisant la méthode de Kjeldahl.

Pour augmenter la charge protéique du milieu biologique, la peptone possède un faible degré d'hydrolyse.

Le pH de la peptone est proche du pH physiologique, c'est-à-dire voisin de 7.

En particulier, la peptone est riche en acides aminés à longue chaîne latérale aromatique ou hydrophobe, tel que la phénylalanine, le tryptophane ou la tyrosine. En effet, ces acides aminés sont la cible préférentielle de la chymotrypsine, l'une des enzymes protéolytiques qui affectent les îlots.

En variante, la peptone est riche en acides aminés carboxyterminaux hydrophobes, tel que la leucine, la phénylalanine et la tyrosine. Ces acides aminés sont reconnus et libérés par la carboxypeptidase.

De façon avantageuse, la peptone est riche en leucine, phénylalanine, tyrosine et tryptophane. Notamment, la teneur cumulée en ces quatre acides aminés dans la peptone est supérieure à 100 mg/g, plus particulièrement 150 mg/g.

De plus, la peptone est choisie avec un taux d'endotoxine le plus faible possible, pour éviter toute contamination. Notamment, le taux d'endotoxine de la peptone est inférieur à 200 EU/g.

La peptone d'origine végétale qui répond aux critères de teneur relativement élevée en leucine, tyrosine, tryptophane et phénylalanine, avec une teneur en protéines supérieure à 70% en poids, un pH physiologique, un faible taux d'hydrolyse et une faible teneur en endotoxine est la peptone de pois ou de pomme de terre. La peptone de pois est préférée. Cette peptone de pois est obtenue par hydrolyse enzymatique des protéines de farine de pois.

Les peptones peuvent être obtenues dans le commerce auprès de divers fabricants et/ou distributeurs tels que Solabia, Millipore, Kerry Biosciences,...

Le milieu biologique contient un ou plusieurs éléments protecteurs à activité antioxydante afin notamment de prévenir les dommages liés à l'hypoxie des cellules bêta qui entraîne la mort cellulaire.

Par exemple, le milieu biologique contient l'antioxydant à base de N-acétylcystéine Stem-ease® (Abcys, France).

Dans un autre exemple, le milieu biologique comprend un cocktail d'éléments protecteurs qui comprend au moins deux capteurs de radicaux libres ou substances antioxydantes. Par exemples, les capteurs de radicaux libres sont choisis parmi le groupe comprenant les dérivés vitaminiques, les dérivés porteurs d'au moins une fonction thiol et les polyols linéaires ou cycliques.

D'autres substances à activité antioxydante sont les dérivés de zinc tel que le sulfate de zinc.

Selon une réalisation particulière, le milieu biologique comprend en outre du polyéthylène glycol, et notamment entre 0,1 et 3% en poids/volume de polyéthylène glycol. Le polyéthylène glycol possède un rôle d'immunoprotecteur.

Une quantité inférieure à 1 % en poids/volume, en particulier 0,5% en poids/volume de polyéthylène glycol dans le milieu biologique est suffisante pour assurer sa fonction d'immunoprotecteur.

Selon une réalisation particulière, le milieu biologique ne contient pas d'hormones, notamment pas de transferrine, ni d'insuline, ni de facteurs de croissance. Les facteurs de croissance utilisés dans les milieux de culture étant généralement des molécules synthétiques complexes et coûteuses, un milieu exempt de telles molécules est donc une alternative particulièrement avantageuse.

Le milieu biologique sans sérum est avantageusement un milieu à usage clinique. Les milieux à usage de recherche sont des milieux utilisés pour cultiver ou conserver des cellules destinées exclusivement pour des études en laboratoire, c'est-à-dire que les cellules ainsi cultivées ne sont pas introduites dans l'homme. Au contraire, un milieu à usage clinique ou thérapeutique ou de grade clinique est utilisé pour cultiver ou conserver des cellules qui seront réimplantées ou réintroduites dans l'homme.

De plus, le milieu est exempt de produit xénogénique, c'est-à-dire appartenant à une autre espèce que l'homme. Il ne contient pas de produit d'origine animale autre que l'homme.

Le milieu est de grade clinique en ce qu'il ne contient pas de produit d'origine animal et contient des éléments acceptés par les autorités réglementaires françaises.

Selon le procédé de l'invention, la préparation de cellules est mise en contact avec le milieu biologique pendant un temps compris entre 1 et 5 jours. Le milieu biologique tel que décrit ci-dessus permet donc de conserver à court terme des cellules sécrétrices d'insuline.

La température de préservation est comprise entre 20°C et 37°C, notamment elle est à température ambiante.

Le milieu biologique du procédé et de la composition de l'invention est prêt-à-l'emploi, conditionné dans un récipient, poche ou flacon. Il ne nécessite pas de reconstitution en laboratoire ou à l'hôpital.

Le milieu contenu dans le récipient est stérile. Il est avantageusement introduit dans le récipient par remplissage aseptique.

Le milieu biologique contenant de l'albumine et au moins une peptone peut également être utilisé lors de l'isolement ou l'extraction des îlots à partir d'un pancréas, notamment à l'étape de rinçage, où le taux d'enzymes exocrines libérées à partir du tissu exocrine digéré est particulièrement important.

Il peut être utilisé comme milieu de transport pour transporter la préparation d'îlots d'un centre à un autre, ou pour la conserver quelques jours. Et il peut également être utilisé pour la greffe d'îlots.

Le procédé de l'invention pour préserver les cellules sécrétrices d'insuline peut donc être mis en oeuvre lors de l'isolement, l'extraction, le rinçage, la culture, la conservation ou le transport des cellules.

Selon un autre aspect, l'invention concerne un médicament pour le traitement des pathologies pancréatiques, tel que le diabète, ledit médicament comprenant une composition telle que définie ci-dessus. La composition comprend notamment de l'albumine dans un produit nutritif et une peptone ou un mélange de peptones.

Cette composition est utilisée pour le traitement des pathologies pancréatiques telles que le diabète. Elle est alors transplantée chez l'homme, par exemple par perfusion.

### Exemple

Des préparations d'îlots ayant une pureté d'environ 40% sont reprises dès la fin de la procédure d'isolement dans trois milieux biologiques différents dans une concentration de 15 000 IEQ/175 cm2/ 30ml. Les préparations d'îlots comprennent environ 3 000 îlots équivalents (IE).

Les îlots sont conservés pendant 5 jours à 37°C, 5% CO2 dans trois milieux différents, renouvelés à 18-24 h.

| | VC | V0 | V1 |
|---|---|---|---|
| Milieu de base | CMRL 1066 | CMRL 1066 | CMRL 1066 |
| PEG (%) | | 0,5 % | 0,5 % |
| Sérum AB (%) | 2,5% | | |
| HSA (%) | | 0,625% | 0,625% |
| Peptone de pois | | | 0,1 % |
| Eléments protecteurs | 0,1 mg/ml Stem-Ease® | Cocktail | Cocktail |

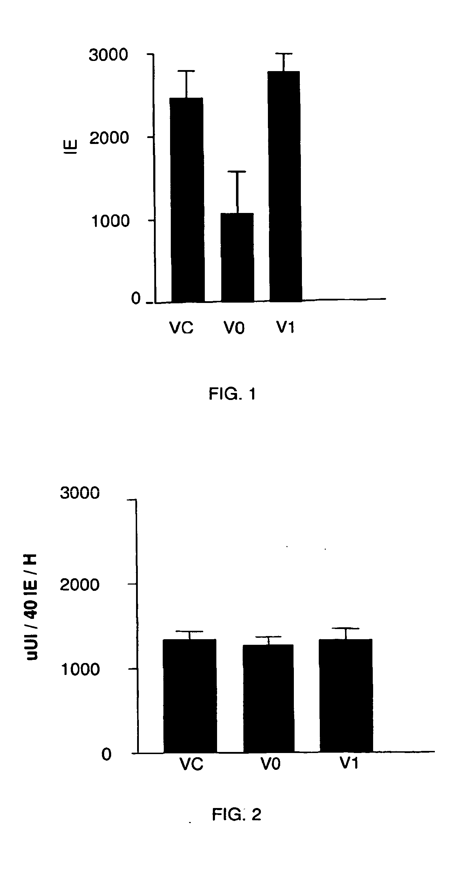

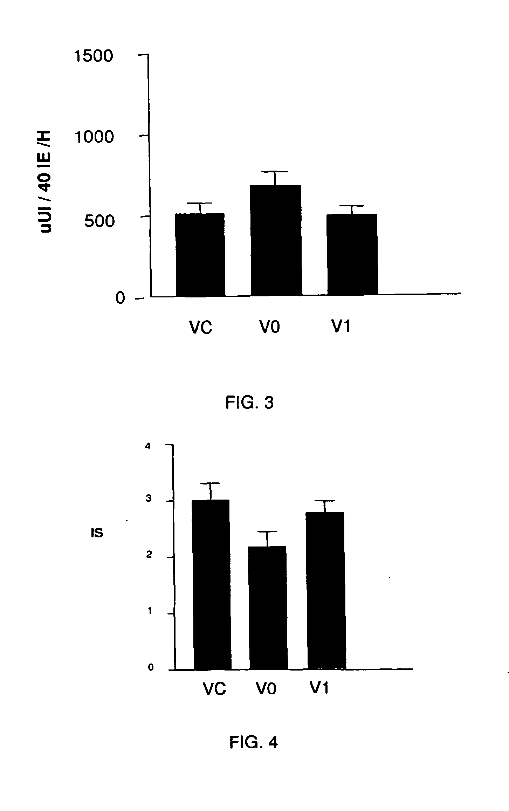

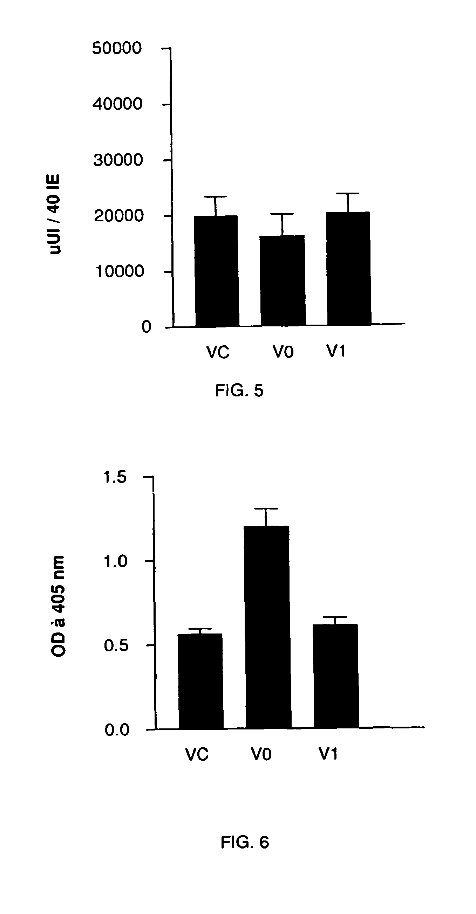

L'évaluation est réalisée au bout du cinquième jour avec les critères suivants :
- le nombre d'îlots équivalents (IE) par comptage des îlots après coloration à la dithizone (figure 1),
- la physiologie des îlots par détermination de la quantité intracellulaire (µIU/40 IE) en insuline (figure 5), du taux de sécrétion d'insuline (µIU/40 IE) en réponse à 20 mM de glucose (figure 2) et à 2,8 mM de glucose (figure 3), et de l'index de stimulation (figure 4),
- la mort cellulaire par dosage (à 405 nm) de l'apoptose (figure 6),

L'estimation microscopique classique de la viabilité, de la purification et du rendement cellulaire a été réalisée par coloration sélective des cellules bêta par la dithizone, chélateur de zinc donc marqueur spécifique des cellules beta, et par le bleu Trypan, colorant d'exclusion qui marque les cellules mortes. Les cellules bêta mortes apparaissent colorées en violet.

L'activité fonctionnelle des cellules bêta a été évaluée par les taux d'insuline intracellulaire et la sécrétion d'insuline en réponse à des tests de stimulation par le glucose.

L'apoptose est caractérisée par la fragmentation de l'ADN en mono et oligonucléosomes qui peuvent être détectés dans les lysats cellulaires. La quantification des nucléosomes a été réalisée à l'aide du kit « Cell Death Detection ELISA » de chez Roche Molecular Biochemicals. Le principe de détection est fondé sur un dosage immunoenzymatique sandwich associant des anticorps monoclonaux dirigés contre l'ADN et les histones. Le dosage a été réalisé sur 3 échantillons de 160 IE par pancréas.

Le contenu en insuline a été dosé par une technique radioimmunologique à l'aide du kit CT de CIS bio international sur les lysats de 3 échantillons de 40 IE par pancréas. Les sécrétions d'insuline ont été estimées pendant 2 périodes consécutives d'une heure. La première en présence de 2,8 mM de glucose et la seconde à la concentration stimulante de 20 mM de glucose. L'index de stimulation est défini comme le rapport des quantités d'insuline sécrétées pendant ces deux périodes (forte sur faible concentration). Les sécrétions d'insuline ont été estimées sur 5 échantillons de 40 IE par pancréas.

Il résulte des essais (figures 1 à 6) que, lorsque le milieu comprend une peptone (V1), il donne de meilleurs résultats que le milieu sans sérum V1 sur tous les paramètres évalués, et est comparable au milieu contrôle (VC) contenant du sérum.

Par conséquent, le milieu V1 comprenant une peptone est une formulation optimisée pour la viabilité (comptage et apoptose) et la production d'insuline (sécrétion d'insuline stimulée par le glucose et insuline intracellulaire).

## Revendications

1. Procédé pour la préservation d'une préparation de cellules sécrétrices d'insuline comprenant l'étape de :
- mettre en contact ladite préparation de cellules sécrétrices d'insuline avec un milieu biologique, ledit milieu biologique comprenant un produit nutritif incluant de l'albumine, **caractérisé en ce que** le milieu biologique comprend en outre une peptone ou un mélange de peptones en quantité suffisante pour préserver les cellules sécrétrices d'insuline.

2. Procédé selon la revendication 1, **caractérisé en ce que** la préparation de cellules sécrétrices d'insuline est une préparation d'îlots pancréatiques.

3. Procédé selon la revendication 2, **caractérisé en ce que** la concentration des îlots pancréatiques dans le milieu biologique est comprise entre 400 et 40 000 îlots équivalents /mL.

4. Procédé selon l'une quelconque des revendications 1 à 3, **caractérisé en ce que** la préparation comprend en outre des enzymes protéolytiques.

5. Procédé selon la revendication 4, **caractérisé en ce que** le milieu biologique comprend un substrat pour lesdites enzymes, ledit substrat incluant ladite peptone.

6. Procédé selon l'une quelconque des revendications 1 à 5, **caractérisé en ce que** le milieu biologique comprend entre 0,1 et 1% en poids/volume d'albumine.

7. Procédé selon l'une quelconque des revendications 1 à 6, **caractérisé en ce que** l'albumine est de l'albumine de sérum humain.

8. Procédé selon l'une quelconque des revendications 1 à 7, **caractérisé en ce que** le milieu biologique comprend entre 0,01 et 10 % en poids/volume de peptone.

9. Procédé selon l'une quelconque des revendications 1 à 8, **caractérisé en ce que** la peptone est une peptone végétale.

10. Procédé selon l'une quelconque des revendications 1 à 9, **caractérisé en ce que** le milieu biologique comprend en outre du polyéthylène glycol.

11. Procédé selon la revendication 10, **caractérisé en ce que** le milieu biologique comprend entre 0,1 et 3% en poids/volume de polyéthylène glycol.

12. Procédé selon l'une quelconque des revendications 1 à 11, **caractérisé en ce que** la préparation de cellules sécrétrices d'insuline est mise en contact avec le milieu biologique pendant un temps compris entre 1 et 5 jours.

13. Procédé selon l'une quelconque des revendications 1 à 12, mis en oeuvre lors de l'isolement, l'extraction, le rinçage, la culture, la conservation ou le transport de cellules sécrétrices d'insuline.

14. Composition obtenue par le procédé selon l'une quelconque des revendications 1 à 13, comprenant (a) une préparation de cellules sécrétrices d'insuline et (b) un milieu biologique avec de l'albumine, et une peptone ou un mélange de peptones.

15. Composition selon la revendication 14 dans laquelle le milieu biologique comprend entre 0,1 et 1% en poids/volume d'albumine, entre 0,01 et 10 % en poids/volume de peptone, et entre 0,1 et 3% en poids/volume de polyéthylène glycol.

16. Médicament pour le traitement des pathologies pancréatiques **caractérisé en ce qu'**il comprend une composition selon la revendication 14 ou 15.

## Patentansprüche

1. Verfahren für die Erhaltung eines Präparats von Insulin-sekretierenden Zellen, umfassend den folgenden Schritt:
- In-Kontakt-Bringen des Präparats von Insulinsekretierenden Zellen mit einem biologischen Medium, wobei das biologische Medium ein Nährprodukt umfasst, darin eingeschlossen Albumin, **dadurch gekennzeichnet, dass** das biologische Medium außerdem ein Pepton oder eine Mischung von Peptonen in ausreichender Menge umfasst, um die Insulin-sekretierenden Zellen zu erhalten.

2. Verfahren nach Anspruch 1, **dadurch gekennzeichnet, dass** das Präparat von Insulin-sekretierenden Zellen ein Präparat von Langerhans-Inseln ist.

3. Verfahren nach Anspruch 2, **dadurch gekennzeichnet, dass** die Konzentration der Langerhans-Inseln im biologischen Medium zwischen 400 und 40000 äquivalenten Inseln /mL liegt.

4. Verfahren nach einem der Ansprüche 1 bis 3, **dadurch gekennzeichnet, dass** das Präparat außerdem proteolytische Enzyme umfasst.

5. Verfahren nach Anspruch 4, **dadurch gekennzeichnet, dass** das biologische Medium ein Substrat für die Enzyme umfasst, wobei das Substrat das Pepton einschließt.

6. Verfahren nach einem der Ansprüche 1 bis 5, **dadurch gekennzeichnet, dass** das biologische Medium zwischen 0,1 und 1 % Gew./Vol. Albumin umfasst.

7. Verfahren nach einem der Ansprüche 1 bis 6, **dadurch gekennzeichnet, dass** das Albumin Albumin von menschlichem Serum ist.

8. Verfahren nach einem der Ansprüche 1 bis 7, **dadurch gekennzeichnet, dass** das biologische Medium zwischen 0,01 und 10 % Gew./Vol. Pepton umfasst.

9. Verfahren nach einem der Ansprüche 1 bis 8, **dadurch gekennzeichnet, dass** das Pepton ein pflanzliches Pepton ist.

10. Verfahren nach einem der Ansprüche 1 bis 9, **dadurch gekennzeichnet, dass** das biologische Medium außerdem Polyethylenglykol umfasst.

11. Verfahren nach Anspruch 10, **dadurch gekennzeichnet, dass** das biologische Medium zwischen 0,1 und 3 % Gew./Vol. Polyethylenglykol umfasst.

12. Verfahren nach einem der Ansprüche 1 bis 11, **dadurch gekennzeichnet, dass** das Präparat von Insulinsekretierenden Zellen mit dem biologischen Medium während eines Zeitraums in Kontakt gebracht wird, der zwischen 1 und 5 Tagen liegt.

13. Verfahren nach einem der Ansprüche 1 bis 12, durchgeführt während der Isolierung, der Extraktion, der Spülung, der Kultivierung, der Erhaltung oder des Transports von Insulin-sekretierenden Zellen.

14. Zusammensetzung, erhalten durch das Verfahren nach einem der Ansprüche 1 bis 13, umfassend (a) ein Präparat von Insulin-sekretierenden Zellen und (b) ein biologisches Medium mit Albumin und ein Pepton oder eine Mischung von Peptonen.

15. Zusammensetzung nach Anspruch 14, wobei das biologische Medium zwischen 0,1 und 1 % Gew./Vol. Albumin umfasst, zwischen 0,01 und 10 % Gew./Vol. Pepton und zwischen 0,1 und 3 % Gew./Vol. Polyethylenglykol.

16. Arzneimittel zur Behandlung von Pankreaserkrankungen, **dadurch gekennzeichnet, dass** es eine Zusammensetzung nach Anspruch 14 oder 15 umfasst.

## Claims

1. Method for preserving a preparation of insulin-secreting cells comprising the step of:
- contacting said preparation of insulin-secreting cells with a biological medium, said biological medium comprising a nutrient product including albumin, **characterised in that** the biological medium further comprises a peptone or a peptone mixture in sufficient quantity to preserve the insulin-secreting cells.

2. Method according to claim 1, **characterised in that** the preparation of insulin-secreting cells is a preparation of pancreatic islets.

3. Method according to claim 2, **characterised in that** the concentration of the pancreatic islets in the biological medium is between 400 and 40,000 equivalent islets/ml.

4. Method according to any of claims 1 to 3, **characterised in that** the preparation further comprises proteolytic enzymes.

5. Method according to claim 4, **characterised in that** the biological medium comprises a substrate for said enzymes, said substrate including said peptone.

6. Method according to any of claims 1 to 5, **characterised in that** the biological medium comprises between 0.1 and 1% by weight/volume of albumin.

7. Method according to any of claims 1 to 6, **characterised in that** the albumin is human serum albumin.

8. Method according to any of claims 1 to 7, **characterised in that** the biological medium comprises between 0.01 and 10% by weight/volume of peptone.

9. Method according to any of claims 1 to 8, **characterised in that** the peptone is a plant peptone.

10. Method according to any of claims 1 to 9, **characterised in that** the biological medium further comprises polyethylene glycol.

11. Method according to claim 10, **characterised in that** the biological medium comprises between 0.1 and 3% by weight/volume of polyethylene glycol.

12. Method according to any of claims 1 to 11, **characterised in that** the preparation of insulin-secreting cells is contacted with the biological medium for a time between 1 and 5 days.

13. Method according to any of claims 1 to 12, used for the isolation, extraction, rinsing, culture, storage or transport of insulin-secreting cells.

14. Composition obtained using the method according to any of claims 1 to 13, comprising (a) a preparation of insulin-secreting cells and (b) a biological medium with albumin, and a peptone or a peptone mixture.

15. Composition according to claim 14 wherein the biological medium comprises between 0.1 and 1% by weight/volume of albumin, between 0.01 and 10% by weight/volume of peptone, and between 0.1 and 3% by weight/volume of polyethylene glycol.

16. Medicinal product for treating pancreatic diseases **characterised in that** it comprises a composition according to claim 14 or 15.
